# EUROPÄISCHE PATENTANMELDUNG

(11) **EP 1 251 119 A2**
(43) Veröffentlichungstag der Anmeldung: **23.10.2002**
(21) Anmeldenummer: 02007355.7
(22) Anmeldetag: 08.04.2002
(51) Int. Cl.: C07C 209/72, C07C 211/36

(54) **Kontinuierliches Verfahren zur Herstellung von Diaminodicyclohexylmethan**

(30) Priorität: 19.04.2001 DE 10119135
(71) Anmelder: BAYER AG, 51368 Leverkusen (DE)
(72) Erfinder: Bunnenberg, Rolf, Dr., 42799 Leichlingen (DE); Gröschl, Andreas, Dr., 51375 Leverkusen (DE); Holzbrecher, Michael, 51766 Engelskirchen (DE); Schulze Tilling , Andreas Dr., League City, TX 77573 (US)

(57) **Zusammenfassung**

Die Erfindung betrifft ein Verfahren zur Herstellung von Diaminodicyclohexylmethan (PACM) mit einem Anteil an trans,trans-4,4'-Diaminodicyclohexylmethan von 17 bis 24 % durch Hydrierung von Diaminodiphenylmethan (MDA) in Gegenwart eines pulverförmigen, insbesondere Ruthenium-haltigen Katalysators, wobei die Hydrierung in einem kontinuierlich betriebenen Suspensionsreaktor durchgeführt wird und der Umsatz an MDA mindestens 95 %, bezogen auf die eingesetzte Menge an MDA beträgt. Vorzugeweise wird in einer Kaskade mehrerer hintereinandergeschalteter Suspensionsreaktoren, beispielsweise einer Rührkesselkaskade oder einer Blasensäulenkaskade gearbeitet.

## Beschreibung

Die vorliegende Erfindung betrifft ein kontinuierliches Verfahren zur Herstellung von Diaminodicyclohexylmethan (PACM) durch Hydrierung von Diaminodiphenylmethan (MDA) in Gegenwart eines pulverförmigen Katalysators.

PACM wird technisch durch Hydrierung von MDA hergestellt. PACM findet beispielsweise Anwendung zur Herstellung von Lacken, hauptsächlich als Vorstufe für den Lackrohstoff Diisocyanatodicyclohexylmethan. Bei einer Reihe von Anwendungen ist das Isomerenverhältnis von besonderer Bedeutung.

Aus EP 639 403 A2 ist ein Katalysator zur Herstellung von PACM mit niedrigem Anteil an trans-trans-Isomer durch Hydrierung von MDA bekannt. Es handelt sich dabei um einen Katalysator mit einer dünnen Ruthenium- oder Rhodium-haltigen Schicht auf einem speziellen Träger, nämlich einer kalzinierten und oberflächlich rehydratisierten Übergangstonerde, insbesondere Hydrargillit oder Bayerit.

EP 639 403 A2 beschreibt die Desaktivierung des Katalysators durch höhermolekulare Bestandteile des Reaktionsgemischs und die Einstellung eines niedrigen Anteils an trans,trans-Isomerem im Produkt als Problem bei der technischen Herstellung von PACM. Durch Verwendung des speziellen Katalysators sollen diese Probleme gelöst werden. Der spezielle Katalysator eignet sich aber hauptsächlich für einen Einsatz in Reaktoren mit einem Katalysatorfestbett, in denen der Katalysator während des Betriebes nicht ausgetauscht werden kann. Zudem wird ein großer Teil des Reaktorvolumens durch den inaktiven Kern des verwendeten Schalenkatalysators ausgefüllt und steht als Reaktionsvolumen nicht mehr zur Verfügung.

Hydrierungen in diskontinuierlich betriebenen Suspensionsreaktoren sind bereits beschrieben. Diese Verfahrensweise hat den Nachteil, dass bei schneller Reaktion am Endpunkt der Reaktion, d.h. bei vollständigem Umsatz und gleichzeitig spezifiziertem Gehalt an trans,trans-Isomerem, die Reaktion nicht schnell genug abgebrochen werden kann. Es besteht also immer die Gefahr, dass man unvollständigen Umsatz oder Produkt mit einem unerwünscht hohen Anteil an trans,trans-Isomer erhält. Man muss daher für diese Reaktionsführung in der Regel Katalysatoren mit geringerer Aktivität einsetzen oder bei niedrigen Temperaturen arbeiten, was zu langen Reaktionszeiten und einer geringen Raum-Zeit-Ausbeute führt.

Aufgabe der Erfindung war es daher, ein kontinuierlich betreibbares Verfahren zur Herstellung von PACM mit einem geringen Anteil an trans,trans-4,4'-Diaminodicyclohexylmethan zur Verfügung zu stellen, das sich durch eine hohe Raum-Zeit-Ausbeute und eine hohe Katalysatorlebensdauer auszeichnet.

Überraschend wurde gefunden, dass die Aufgabe gelöst werden kann, indem man die Hydrierung von MDA zu PACM in einem kontinuierlich betriebenen Suspensionsreaktor durchführt.

Gegenstand der Erfindung ist ein Verfahren zur Herstellung von Diaminodicyclohexylmethan (PACM) mit einem Anteil an trans,trans-4,4'-Diaminodicyclohexylmethan von 17 bis 24 % durch Hydrierung von Diaminodiphenylmethan (MDA) in Gegenwart eines pulverförmigen Katalysators, das dadurch gekennzeichnet ist, dass die Hydrierung in einem kontinuierlich betriebenen Suspensionsreaktor durchgeführt wird und der Umsatz an MDA mindestens 95 %, bezogen auf die eingesetzte Menge an MDA beträgt.

Vorzugsweise beträgt der Umsatz an MDA mindestens 99 %, bezogen auf die eingesetzte Menge an MDA. Der Umsatz lässt sich durch dem Fachmann bekannte Maßnahmen, beispielsweise durch Einstellung der Verweilzeit im kontinuierlich betriebenen Suspensionsreaktor beeinflussen.

Besonders vorteilhaft ist es, in einer Kaskade mehrerer hintereinandergeschalteter Suspensionsreaktoren zu arbeiten, beispielsweise in einer Rührkesselkaskade oder einer Blasensäulenkaskade. Vorzugsweise wird in einer Kaskade mehrerer hintereinandergeschalteter Suspensionsreaktoren gearbeitet, die aus mindestens drei hintereinandergeschalteten Reaktoren besteht.

Die Vermischung von Edukt (MDA) mit dem eingesetzten pulverförmigen Katalysator und dem zur Hydrierung benötigten Wasserstoff erfolgt bei Verwendung von Rührkesseln als Suspensionsreaktoren mittels eines Rührers, bei Verwendung von Blasensäulen als Suspensionsreaktoren durch den Eintrag von Wasserstoff mit hoher Geschwindigkeit und die Erzeugung einer turbulenten Strömung im Reaktor.

Der erfindungsgemäß einzusetzende pulverförmige Katalysator enthält vorzugsweise Ruthenium, vorzugsweise 1 bis 10 Gew.-%, besonders bevorzugt 4 bis 8 Gew.-% Ruthenium.

Vorzugsweise ist das Ruthenium in feiner Verteilung auf einen Träger aufgebracht, um eine gute Katalysatorlebensdauer und gute Filtrierbarkeit zu gewährleisten. Geeignete Träger sind beispielsweise Aluminiumoxide.

In einer besonderen Ausführungsform ist das Ruthenium weitgehend homogen über den Querschnitt der Trägerpartikel verteilt. Hierdurch wird gewährleistet, dass bei der mechanischen Belastung im Reaktor keine Rutheniumpartikel von dem Träger abgelöst werden, was bei Schalenkatalysatoren leicht möglich ist. Die mechanische Beanspruchung im Reaktor hat teilweise den Bruch der Katalysatorkörner zur Folge. Dadurch entsteht beim homogen imprägnierten Katalysator im Gegensatz zu einem Schalenkatalysator keine rutheniumfreie Oberfläche, sondern eine frische, aktive rutheniumhaltige Katalysatoroberfläche.

Ein Katalysator bei dem das Ruthenium weitgehend homogen über den Querschnitt der Trägerpartikel verteilt ist, kann beispielsweise hergestellt werden, indem man zunächst eine wässrige Lösung eines Rutheniumsalzes, z.B. Rutheniumchlorid oder Rutheniumnitrosylnitrat auf ein Aluminiumoxidpulver einwirken lässt und das Ruthenium anschließend durch die Zugabe einer Base, z.B. NaOH ausfällt.

Vorzugsweise wird der Katalysator als Pulver mit einem mittleren Durchmesser der Katalysatorkörner von 5 bis 150 µm, besonders bevorzugt 10 bis 120 µm, insbesondere bevorzugt 30 bis 100 um eingesetzt.

Der Katalysator kann beispielsweise in einer Menge von 1 bis 10 Gew.-%, bevorzugt 3 bis 8 Gew.-%, bezogen auf die Reaktionsmischung eingesetzt werden.

Das erfindungsgemäße Verfahren wird beispielsweise bei einer Temperatur von 130 bis 200°C, bevorzugt von 140 bis 190°C durchgeführt, besonders bevorzugt von 150 bis 180 °C.

Beim Einsatz eines Kaskadenreaktors können die Temperaturen der einzelnen Reaktoren unterschiedlich sein. Es ist vorteilhaft die Temperatur im ersten Reaktor höher zu wählen, als im letzten Reaktor. Bei einer Kaskade aus drei Reaktoren kann man beispielsweise den ersten Reaktor bei 180 °C, den zweiten bei 170 °C und den dritten Reaktor bei 150 °C betreiben.

Der Wasserstoffdruck beträgt beispielsweise von 50 bis 400 bar, bevorzugt von 100 bis 200 bar.

Wasserstoff wird vorteilhaft in einem Überschuss von 5 bis 200 %, bevorzugt von 20 bis 100 % der Theorie zugegeben.

Das erfindungsgemäße Verfahren kann mit und ohne den Zusatz von organischen Lösungsmitteln durchgeführt werden. Als Lösungsmittel eignen sich beispielsweise Alkohole, bevorzugt sekundäre Alkohole, z.B. Isobutanol, Cyclohexanol oder Methylcyclohexanol oder tertiäre Alkohole, z.B. tert.-Butanol, besonders bevorzugt tertiäre Alkohole.

Das Lösungsmittel kann nach Abtrennung des Katalysators destillativ vom Produkt getrennt und in den Hydrierprozess zurückgeführt werden.

Es ist vorteilhaft, den Gehalt an Wasser im Reaktionsgemisch klein zu halten, da Wasser eine Desaktivierung des Katalysators zur Folge hat. Bevorzugt wird der Anteil von Wasser im Reaktionsgemisch kleiner als 1 Gew.-%, besonders bevorzugt kleiner als 0,5 Gew.-% gehalten.

Im erfindungsgemäßen Verfahren kann Diaminodiphenylmethan (MDA) eingesetzt werden, das neben MDA höhermolekulare aromatische Amine enthält.

Um eine optimale Raum-Zeit-Ausbeute zu erzielen, ist es vorteilhaft, das Reaktionsgemisch vor Einspeisung in den kontinuierlich betriebenen Suspensionsreaktor auf die Reaktionstemperatur zu bringen.

Mittels der Parameter Katalysatorkonzentration, Temperatur und Verweilzeit im Reaktor kann der Gehalt an trans,trans-Isomer im Produkt eingestellt werden. So sind Produkte mit einem niedrigen Anteil an trans,trans-Isomer, insbesondere mit einem Anteil zwischen 17 und 24 % zu erreichen. Beispielsweise kann bei gegebener Temperatur und Katalysatorkonzentration durch Anpassen der Verweilzeit der Reaktionsmischung im Reaktor der Anteil an trans,trans-Isomer im Produkt eingestellt werden.

Beim erfindungsgemäßen Verfahren kann der Katalysator zusammen mit dem Reaktionsgemisch durch den Reaktor oder die Reaktorkaskade gefördert werden. Anschließend wird die Produktmischung in der Regel abgekühlt, der überschüssige Wasserstoff abgeschieden und der Katalysator abfiltriert.

Vorzugsweise wird der Katalysator nach der Abtrennung der Produktlösung erneut eingesetzt.

Es ist bezüglich der Katalysatoraktivität und der Lebensdauer vorteilhaft, den Katalysator nach der Abtrennung der Produktlösung mit einem Lösungsmittel zu waschen. Hierdurch kann die Katalysatoroberfläche von Ablagerungen höhermolekularer Reaktionsprodukte befreit werden.

Bei abfallender Katalysatoraktivität nach längerem Betrieb können Teilmengen des Katalysators ausgeschleust und durch frischen Katalysator ersetzt werden, so dass eine Anlage zur Durchführung des erfindungsgemäßen Verfahrens mit konstanter mittlerer Katalysatoraktivität und konstantem Durchsatz gefahren werden kann.

Im folgenden wird die Erfindung anhand von Beispielen weiter erläutert. Die Beispiele stellen einzelne Ausführungsformen der Erfindung dar, die Erfindung ist jedoch nicht auf die Beispiele beschränkt.

### Beispiel 1

Der Versuch wurde in einem kontinuierlich betriebenen Rührkessel durchgeführt. Das Reaktionsvolumen betrug 330 ml; es wurde ein pulverförmiger Katalysator mit 5 Gew.-% Ruthenium auf einem Al₂O₃-Träger in einer Katalysatorkonzentration von 5 Gew.-% in dem Rührkessel vorgelegt. MDA wurde in technischer Qualität (Bezeichnung MDA 90/10) mit einem Anteil von ca. 10 % an höhermolekularen Komponenten als 33 Gew.-%ige Lösung in Isobutanol eingesetzt. Das MDA 90/10-Isobutanol-Gemisch wurde aus einem Vorlagenbehälter in den Reaktor dosiert. Der Reaktordruck wurde durch ständiges Nachführen von Wasserstoff auf 150 bar konstant gehalten. Bei dem Versuch wurde eine Temperatur von 150°C eingestellt.

Der Überlauf des Reaktionsgemisches erfolgte über einen weiteren Behälter, aus dem Proben zur Analyse gezogen wurden. Die Proben wurden mittels Gaschromatographie analysiert. Durch Variation der Förderleistung der Dosierpumpe wurden verschiedene mittlere Verweilzeiten eingestellt. Zum Vergleich wurde die Verweilzeit in einem Fall so eingestellt, dass der Umsatz an MDA, bezogen auf die Menge an eingesetztem MDA lediglich 91,5 % betrug. In diesem Fall lag der Anteil an trans,trans-Isomer des erhaltenen PACM unterhalb des gewünschten Bereichs.

Die Gehalte an MDA, [H₆]-MDA (4-Aminocyclohexyl-4-aminophenylmethan), PACM und der Anteil an trans,trans-PACM (t,t-Anteil) sind in Tabelle 1 wiedergegeben.

| | **Einstufiger Rührkessel** | **Einstufiger Rührkessel** | **Einstufiger Rührkessel (Vergleich)** |
|---|---|---|---|
| Temperatur [°C] | 150 | 150 | 150 |
| Verweilzeit [min] | 61 | 29 | 12 |
| Durchsatz [g(PACM) pro 1 und h] | 247 | 494 | 715 |
| PACM [%] | 88 | 81 | 48 |
| tt-Anteil [%] | 24 | 17 | 14 |
| [H₆]-MDA [%] | 1,5 | 8,2 | 34 |
| MDA [%] | 0,1 | 0,7 | 8,5 |
| Höhermolekulare [%] | 10 | 10 | 10 |

### Beispiel 2

Der Versuch wurde unter den gleichen Bedingungen wie in Beispiel 1 durchgeführt, allerdings wurde die Verweilzeit so gekürzt, dass nur Teilumsatz erfolgte. Anschließend wurde das Produkt zwei weitere Male durch den Reaktor gefördert. Das Produkt entspricht dem Produkt, das in einer Kaskade aus drei Rührkesseln erhalten wird.

Die Gehalte an MDA, [H₆]-MDA, PACM und der Anteil an trans,trans-PACM (t,t-Anteil) sind in Tabelle 2 wiedergegeben.

| | **Einstufiger Rührkessel** | **Dreistufiger Rührkessel** |
|---|---|---|
| Temperatur [°C] | 150 | 150 |
| Verweilzeit [min] | 51 | 45 |
| Durchsatz [g(PACM) pro 1 und h] | 247 | 330 |
| PACM [%] | 88 | 89 |
| tt-Anteil [%] | 24 | 19 |
| [H₆]-MDA [%] | 1,5 | 0,9 |
| MDA [%] | 0,1 | 0,1 |
| Höhermolekulare [%] | 10 | 10 |

Der Versuch zeigt, dass bei Einsatz einer Kaskade von drei Reaktoren gleichzeitig sehr hoher Umsatz von MDA und ein trans,trans-Anteil im Bereich von 20 % erzielt werden kann und zugleich eine hohe Raum-Zeit-Ausbeute erreicht wird.

### Vergleichsbeispiel: Diskontinuierlich betriebener Rührkessel

Der Versuch wurde in einem diskontinuierlich betriebenen Rührkessel durchgerührt. Das Reaktionsvolumen betrug 330 ml; es wurde ein pulverförmiger Katalysator mit 5 Gew.-% Ruthenium auf einem Al₂O₃-Träger in einer Katalysatorkonzentration von 5 Gew.-% in den Rührkessel vorgelegt. MDA wurde in technischer Qualität (Bezeichnung MDA 90/10) mit einem Anteil von ca. 10 % an höhermolekularen Komponenten als 33 gew.-%ige Lösung in Isobutanol eingesetzt. 330 ml des MDA 90/10-Isobutanol-Gemischs wurden aus einem Vorlagenbehälter in den Reaktor dosiert. Der Reaktordruck wurde durch ständiges Nachführen von Wasserstoff auf 150 bar konstant gehalten. Bei dem Versuch wurde eine Temperatur von 150°C eingestellt.

Aus dem Reaktionsgemisch wurden nach unterschiedlicher Verweilzeit des Reaktionsgemischs im Reaktor Proben zur Analyse gezogen. Die gezogenen Proben wurden mittels Gaschromatographie analysiert.

Die Gehalte an MDA, [H₆]-MDA, PACM und der Anteil an trans,trans-PACM (t,t-Anteil) sind in Tabelle 3 wiedergegeben.

| | **Diskontinuierlicher Rührkessel** | **Diskontinuierlicher Rührkessel** | **Diskontinuierlicher Rührkessel** |
|---|---|---|---|
| Temperatur [°C] | 150 | 150 | 150 |
| Verweilzeit [min] | 51 | 111 | 171 |
| Durchsatz [g(PACM) pro 1 und h] | 282 | 131 | 81 |
| PACM [%] | 84 | 85 | 81 |
| tt-Anteil [%] | 14 | 24 | 37 |
| [H₆]-MDA [%] | 3,1 | 0,2 | 0,3 |
| MDA [%] | 0,5 | 0,1 | 0 |
| Höhermolekulare [%] | 12 | 14 | 18 |

Der Versuch zeigt, dass Produkt mit einem Anteil von 20 % trans,trans-Isomer nur bei wesentlich geringerer Raum-Zeit-Ausbeute als bei kontinierlicher Hydrierung in einer Kaskade aus drei Reaktoren erhalten werden kann.

## Patentansprüche

1. Verfahren zur Herstellung von Diaminodicyclohexylmethan (PACM) mit einem Anteil an trans,trans-4,4'-Diaminodicyclohexylmethan von 17 bis 24 % durch Hydrierung von Diaminodiphenylmethan (MDA) in Gegenwart eines pulverförmigen Katalysators, **dadurch gekennzeichnet, dass** die Hydrierung in einem kontinuierlich betriebenen Suspensionsreaktor durchgeführt wird und der Umsatz an MDA mindestens 95 %, bezogen auf die eingesetzte Menge an MDA beträgt.

2. Verfahren gemäß Anspruch 1, **dadurch gekennzeichnet, dass** als kontinuierlich betriebener Suspensionsreaktor eine Kaskade mehrerer hintereinandergeschalteter Suspensionsreaktoren eingesetzt wird.

3. Verfahren gemäß Anspruch 2, **dadurch gekennzeichnet, dass** es sich bei der Kaskade mehrerer hintereinandergeschalteter Suspensionsreaktoren um eine Rührkesselkaskade handelt.

4. Verfahren gemäß Anspruch 2, **dadurch gekennzeichnet, dass** es sich bei der Kaskade mehrerer hintereinandergeschalteter Suspensionsreaktoren um eine Blasensäulenkaskade handelt.

5. Verfahren gemäß wenigstens einem der Ansprüche 1 bis 4, **dadurch gekennzeichnet, dass** der eingesetzte pulverförmige Katalysator 1 bis 10 Gew.-% Ruthenium enthält.

6. Verfahren gemäß Anspruch 5, **dadurch gekennzeichnet, dass** das Ruthenium auf einen Träger aufgebracht und über den gesamten Querschnitt der Trägerpartikel verteilt ist.

7. Verfahren gemäß wenigstens einem der Ansprüche 1 bis 6, **dadurch gekennzeichnet, dass** der Katalysator als Pulver mit einem mittleren Durchmesser von 5 bis 150 um eingesetzt wird.

8. Verfahren gemäß wenigstens einem der Ansprüche 1 bis 7, **dadurch gekennzeichnet, dass** bei einer Temperatur von 130 bis 200°C gearbeitet wird.

9. Verfahren gemäß wenigstens einem der Ansprüche 1 bis 8, **dadurch gekennzeichnet, dass** bei einem Druck von 50 bis 400 bar gearbeitet wird.

10. Verfahren gemäß wenigstens einem der Ansprüche 1 bis 9, **dadurch gekennzeichnet, dass** die Hydrierung in Gegenwart eines Alkohols als Lösungsmittel durchgeführt wird.

11. Verfahren gemäß wenigstens einem der Ansprüche 1 bis 10, **dadurch gekennzeichnet, dass** der Anteil von Wasser im Reaktionsgemisch kleiner als 1 Gew.-% ist.

12. Verfahren gemäß wenigstens einem der Ansprüche 1 bis 11, **dadurch gekennzeichnet, dass** Diaminodiphenylmethan (MDA) eingesetzt wird, das neben MDA höhermolekulare aromatische Amine enthält.

13. Verfahren gemäß wenigstens einem der Ansprüche 1 bis 12, **dadurch gekennzeichnet, dass** der pulverförmige Katalysator im Diaminodiphenylmethan suspendiert wird und die Mischung vor Einspeisung in den kontinuierlich betriebenen Suspensionsreaktor auf die Reaktionstemperatur gebracht wird.

14. Verfahren gemäß wenigstens einem der Ansprüche 1 bis 13, **dadurch gekennzeichnet, dass** die Produktlösung vom Katalysator abgetrennt wird und der Katalysator nach der Abtrennung der Produktlösung mit einem Lösungsmittel gewaschen wird.

15. Verfahren gemäß wenigstens einem der Ansprüche 1 bis 14, **dadurch gekennzeichnet, dass** die Produktlösung vom Katalysator abgetrennt wird und der Katalysator nach der Abtrennung der Produktlösung erneut eingesetzt wird.
